# EUROPEAN PATENT APPLICATION

(11) **EP 2 441 442 A1**
(43) Date of publication of application: **18.04.2012**
(21) Application number: 10013439.4
(22) Date of filing: 08.10.2010
(51) Int. Cl.: A61K 9/16, A61K 9/20, A61K 9/28, A61K 31/155, A61K 31/4439

(54) **A pharmaceutical composition comprising a thiazolidinedione**

(71) Applicant: LEK Pharmaceuticals d.d., 1526 Ljubljana (SI)
(72) Inventor: The designation of the inventor has not yet been filed
(74) Representative: Hodzar, Damjan

(57) **Abstract**

A pharmaceutical composition comprises a combination of thiazolidinedione or a pharmaceutically acceptable salt thereof as a first pharmaceutically active ingredient and a second pharmaceutically active ingredient different from thiazolidinedione, wherein the amount of said second pharmaceutically ingredient is larger than that of the first pharmaceutically active ingredient, and wherein the combination of said first and second pharmaceutically active ingredients are provided by a first granulate comprising the first and second pharmaceutically active ingredients and optionally at least one excipient, said first granulate being present in a second granulate comprising a further pharmaceutical excipient.

## Description

### FIELD OF THE INVENTION

The present invention relates to a pharmaceutical composition comprising a thiazolidinedione in combination with a second pharmaceutically active ingredient, the process of manufacturing the composition and its use as a medicament in mammals.

### BACKGROUND OF THE INVENTION

The primary goal in treatment of diabetes mellitus is to maintain blood glucose levels as constant as possible. This is critical not only to avoid serious immediate complications like diabetic shock or coma, but also to reduce the risk of extremely dangerous consequences that abnormal blood glucose levels have on the long run. Diabetes can cause damage in small vessels, which results in serious complications such as retinopathy, neuropathy, nephropathy and a dramatically elevated cardiovascular risk in general.

Diabetes patients are therefore generally obliged to observe strict rules regarding their diet in order to keep blood glucose levels in a normal range. As this by itself often proves to be insufficient, however, many diabetes patients need additional pharmaceutical treatment. Several substance classes acting via different mechanisms have been developed, many of which are used routinely as approved medicines; nevertheless research in the field continues and new compounds as well as new formulations are constantly being tested for their clinical effectiveness and safety.

Thiazolidinediones represent an important class of orally administered diabetic drugs that exert their effect by Increasing the cells' insuline sensitivity. Another important group of substances for oral administration are biguanide derivatives. Biguanides, such as metformin lead to reduced plasma glucose levels by various mechanisms, which are not fully understood. However, it appears that both, reduction of hepatic glucose production as well as increased insulin sensitivity through elevated expression of insuline receptors on muscle cells are involved. For increased effectiveness, these substances may also be used in combination.

When formulating solid dosage forms comprising a thiazolidinedione in combination with, for example, a biguanide, several technical difficulties have to be overcome. Thiazolidinediones are usually applied in doses, that are much lower than those of biguanides. Therefore, it is crucial to avoid segregation of the low dose active ingredient and to obtain uniform tabletting mixtures and dosage forms in order to ensure the quality and safety of the drug throughout a given production batch.

Furthermore, complete dissolution of thiazolidinediones, which usually are less soluble than biguanides, is often an issue with such compositions.

Depending on the type of pharmaceutical composition the particle sizes of the two pharmaceutical active ingredients will have an impact on the complications above.

The technically least sophisticated approach is to blend all the ingredients as dry powders and tablet them by direct compression. This, however, is rarely successful for low dose drugs, a common problem being segregation of the powder blend during tabletting.

Hence, compositions have been designed as, for example, in WO01/35941, which employ separate granulates comprising each of the active ingredients that are then compressed together. EP1561472A1 describes a solid preparation having a phase, in which a thiazolidinedione and metformin are uniformly dispersed. Uniform dispersion in said patent application is obtained within a certain ratio of particle size of two active substances. Also the compositions described In WO09/136884 depend on thiazolinedione particle size.

Especially when using materials that do not fulfill the particle size requirements, uniformity cannot be ensured, let alone proper dissolution of the pharmaceutically active ingredients.

It is therefore an objective of the present invention to provide an improved pharmaceutical composition containing thiazolidinone and another pharmaceutically active ingredient that is present larger amount than the thiazolidinone, especially in view of uniformity and release characteristics.

### SUMMARY OF THE INVENTION

The present invention provides the following aspects, subject-matters and preferred embodiments, which respectively taken alone or in combination, further contribute to solving the object of the present invention:
(1) A pharmaceutical composition comprising a combination of thiazolidinedione or a pharmaceutically acceptable salt thereof as a first pharmaceutically active ingredient and a second pharmaceutically active ingredient different from thiazolidinedione, wherein the amount of said second pharmaceutically ingredient is larger than that of the first pharmaceutically active ingredient, wherein the combination of said first and second pharmaceutically active ingredients are provided by a first granulate comprising the first and second pharmaceutically active ingredients and optionally at least one excipient, said first granulate being present in a second granulate comprising a further pharmaceutical excipient.
   The provision of the combination of active ingredients in the above mentioned first granulate being present in the second granulate comprising a further pharmaceutical excipient resembles a structure which can be achieved by subjecting said first granulate to a further granulation step (two-step granulation). This final granulate can then either be directly used in the form of granules to be filled in sachets or capsules, or be formulated into a desired solid dosage form, e.g. compressed optionally with additional excipients into tablets or processed otherwise into a final dosage form.
   The composition of the present invention is different from structures that are respectively obtained when using merely conventional blending of ingredients, or merely using one-step granulation and optional further processing, or merely mixing and compressing of granulates each containing respective active ingredients and excipients.
(2) The pharmaceutical composition according to item 1, wherein the second granulate is in the form of tablets or tablet cores.
(3) The pharmaceutical composition according to item 1, wherein the second granulate is filled into capsules or sachets.
(4) The pharmaceutical composition according to item 2, wherein the tablets or tablet cores optionally comprise a coat.
(5) The pharmaceutical composition according to item 4, wherein the coat comprises one or more substances selected from the group consisting of hydroxpropylcellulose, polyethylene glycol, titanium dioxide and talc.
(6) The pharmaceutical composition according to any of the preceding items, wherein the pharmaceutically active ingredient different from thiazolidinedione is a biguanide derivative.
(7) The pharmaceutical composition according to any of the preceding items, wherein the pharmaceutically active ingredient different from thiazolidinedione is metformin hydrochloride.
(8) The pharmaceutical composition according to any of the preceding items, wherein the thiazolidinedione is pioglitazone or a pharmaceutically active salt thereof.
(9) The pharmaceutical composition according to item 2, wherein the first and second pharmaceutically active ingredients are uniformly distributed throughout the tablet or tablet core.
(10) The pharmaceutical composition according to any of the preceding items, wherein the drug load of the thiazolidinedione per dosage unit is 5 wt.-% of below, optionally 3 wt.-% or below.
(11) The pharmaceutical composition according to any of the preceding items, wherein the amount of the second pharmaceutically active ingredient is 10-fold higher or more, optionally 25-fold higher or more than the amount of the thiazolidinedione.
(12) The pharmaceutical composition according to any of the preceding items, wherein the amount of thiazolidinedione or a pharmaceutically acceptable salt thereof is in the range from 0.01 mg to 500 mg per dosage unit, preferably from 1 mg to 100 mg per dosage unit, and the amount of the second pharmaceutically active ingredient is in the range from 100 mg to 3000 mg per dosage unit, preferably from 250 mg to 2500 mg per dosage unit.
(13) The pharmaceutical composition according to any of the preceding items, wherein the ratio of median size of the thiazolidinedione compound, preferably when the thiazolidinedione compound is pioglitazone hydrochloride, to the median size of the second pharmaceutically active ingredient, preferably when it is metformin hydrochloride, is higher than 15.
(14) The pharmaceutical composition according to any of the preceding items, wherein the thiazolidinedione compound, preferably the pioglitazone hydrochloride, has been added to the composition as micronized powder, and the second pharmaceutically active ingredient, preferably metformin hydrochloride, has been added as coarse particles.
(15) The pharmaceutical composition according to item 14, wherein the micronized powder of the thiazolidinedione compound has a size of d0,5 of below 5µm and d0,9 of below 10µm, and wherein the coarse particies of the second pharmaceutically active ingredient have a size of d0,9 of above 250µm.
(16) The pharmaceutical composition according to any of the preceding items, which comprises povidone, hypromelose and/or hydroxypropyl cellulose as a binder.
(17) The pharmaceutical composition according to item 8 wherein the *in vitro* dissolution rate of pioglitazone as determined by USP 711 is such that after 30 minutes, preferably after 20 minutes, more than 80 % of the total amount of ploglitazone is dissolved.
(18) The pharmaceutical composition according to any of the preceding Items characterized by a hardness higher than 200N and friability below 0,2% at 15/850 strength, and hardness higher than 150N and friability below 0,2% at 15/500 strength.
(19) A process for manufacturing a pharmaceutical composition comprising a combination of thiazolidinedione or a pharmaceutically acceptable salt thereof as a first pharmaceutically active ingredient and a second pharmaceutically active ingredient different from thiazolidinedione, the process comprising the steps of
   a) granulation of the first and second pharmaceutically active ingredients together with at least one suitable excipient to obtain a first granulate, and
   b) subsequent granulation of the first granulate of step a), together with the same or different at least one pharmaceutical excipient.
(20) The process according to item (19), wherein the first granulation step is carried out in a high shear granulator, and the second granulation step is carried out in a fluid bed device.
(21) The process according to item (19) or (20), comprising the steps of
   a) wet granulation of the pharmaceutically active ingredients and optionally at least one suitable excipient;
   b) drying the granulate obtained in step a);
   c) optionally sieving the granulate from step b);
   d) wet granulation of the granulate from step b) or c), together with a granulation liquid comprising the same or different at least one pharmaceutical excipient;
   e) drying the granulate obtained in step d) and
   f) optionally sieving the granulate from step e).
(22) The pharmaceutical composition according to any of items (1) to (18) for use as a medicament for prophylaxis or treatment of diabetes and/or diabetic complications.

### BRIEF DESCRIPTION OF THE DRAWINGS

- **Figure 1:**: Dissolution profiles of tablets prepared in Example 1 and Comparative Examples 1 and 2. Tablets have been analyzed following the protocol according to the USP711, using Apparatus 2 at 50 rpm, 37°C, pH 2.5.
- **Figure 2:**: Contents of active ingredients per tablet (core) in percent of desired total amount (Example 1)
- **Figure 3:**: Dissolution profile of tablets prepared in Example 2. Tablets have been analyzed following the protocol according to the USP711, using Apparatus 2 at 50 rpm, 37°C, pH 2.5.
- **Figure 4:**: Contents of active ingredients per tablet (core) in percent of desired total amount (Example 2)
- **Figure 5:**: Contents of active ingredients per tablet (core) in percent of desired total amount (Comparative Example 1)
- **Figure 6:**: Contents of active ingredients per tablet (core) in percent of desired total amount (Comparative Example 2)
- **Figure 7:**: microscopic illustrations of samples that have been obtained by three different techniques, including dry mixture of ingredients (sample a; Comparative Example 3), high-shear granulation (sample b; Comparative Example 4), and two-step granulation (sample c; Example 3).

### DESCRIPTION OF THE PREFERRED EMBODIMENTS/GENERAL DESCRIPTION

The present invention is now described in more detail by preferred embodiments and examples, which are however presented for illustrative purpose only and shall not be understood as limiting the scope of the present invention in any way.

According to the present invention a pharmaceutical composition comprising a thiazolidinedione or a pharmaceutically acceptable salt thereof (first pharmaceutically active ingredient) and another (different second) pharmaceutically active ingredient is provided. As outlined above, a thiazolidinedione is combined, for example, with a biguanide such as metformin. In such formulations the thiazolidinedione is typically present in amounts significantly lower than those of metformin, which generally renders formulation of uniform pharmaceutical compositions difficult.

It has surprisingly been found that solid dosage forms of excellent uniformity and showing improved dissolution can be obtained when a thiazolidinedione or a pharmaceutically acceptable salt thereof and another pharmaceutically active ingredient are formulated together in a two-step granulation process. Uniform structure and excellent active ingredient homogeneity of the composition of the present invention is achieved by a first granulation step, in which thiazolidinedione as the first pharmaceutically active ingredient is granulated together with a second pharmaceutically active ingredient and at least one suitable pharmaceutical excipient, which is followed by a second granulation step, in which the granulate obtained in the first granulation step is granulated together with the same at least one pharmaceutical excipient as in the first granulation step or with a different at least one excipient. It is believed that incorporating first granulate, having both active ingredients in it, in a second granulate efficiently counteracts segregation, yet can achieve good dissolution and disintegration properties. At the same time, final dosage forms such as tablets achieve improved hardness and prolonged friability.

Unexpectedly, the compositions of the present invention are characterized by excellent uniformity, notwithstanding the relatively simple manufacturing process. Analysis have demonstrated that even when low amounts of pharmaceutically active ingredients are used, the formulation of the present invention does not show a tendency of segregation.

Since the present invention allows to efficiently avoid segregation tendency, appropriate content uniformity of obtained solid dosage forms is significantly enhanced, thereby assuring uniform contents in desired dosage forms such as tablet cores, tablets, and granules filled in capsules or sachets. This is significant given the different contents of first and second pharmaceutical ingredients, especially when, as the case may be, the contents are substantially different and, even more, when the thiazolidinedione as the first pharmaceutically active ingredient is present in a particle size substantially smaller than the second pharmaceutically active ingredient. The advantages of the present invention are even more significant, as the problem of homogeneity is even more likely to occur when two active substances are incorporated in one final dosage form, especially when two actives are very different regarding the dose and particle size. Improved content uniformity efficiently contributes to a marked increase in bioavailability. Improved content uniformity also favors to avoid toxicity in the otherwise possible event that the amount of drug substance would be too high.

The structure of the pharmaceutical composition according to the present invention cannot be obtained either by a process including only dry mixing, dry blending or dry granulation in one step, nor by one-step wet granulation.

While the present invention provides the advantage that relevant properties such as uniformity and dissolution properties are less prone to influences of substantially different sizes of the respective active ingredients, in preferred embodiments the starting material of the thiazolidinedione compound is used as micronized powder, and the starting material of the second pharmaceutically active ingredient is used as relatively coarser particles, preferably without the need of being milled or micronized, and both are granulated together in the two steps as disclosed herein. The micronized powder of the thiazolidinedione compound preferably has a size of d0,5 of below 5µm (In particular d0,50 2-5µm) and d0,9 of below 10µm, and the coarse particles of the second pharmaceutically active ingredient preferably have a size of d0,9 of above 250µm. This embodiment is particularly advantageous when pioglitazone is the thiazolidinedione compound and metformin is the second pharmaceutically active ingredient. Since pioglitazone hydrochloride is a substance poorly soluble in aqueous media, it is desirable that it is micronised so that it is dissolved and absorbed in appropriate amount. On the other hand, since most of available metformin hydrochloride has large particle size and that the milling of metformin hydrochloride is time consumptive, this invention provides an unnecessity of milling and an independency of metformin hydrochloride particle size. Despite of such size difference between the two active ingredients, uniformity and control against segregation is still achieved according to the present invention. In principle, similar observations apply when a thiazolidinedione compound other than pioglitazone hydrochloride, and when, depending on the desire, a second pharmaceutically active ingredient other than metformin are used. The uniform dispersion of both actives in the solid preparation according to the present invention is achieved both, when the ratio of median size of the thiazolidinedione compound (preferably pioglitazone hydrochloride) to the median size of the second pharmaceutically active ingredient (preferably metformin hydrochloride) is up to 15, and also when this ratio Is higher than 15. Unexpectedly and advantageously, excellent combination of properties including dissolution rates and content uniformity of metformin and content uniformity of the thiazolidinone component are independent from particle sizes.

It has furthermore been found that the solid dosage forms of the present invention readily dissolve and thus provide for good bioavailability. This is especially critical for the first thiazolidinedione ingredient, as it is present in combination with the second pharmaceutically active ingredient in the solid dosage form in relatively lower content, typically in substantially lower content. The *in vitro* dissolution rates have been measured for the pharmaceutically active ingredients using Apparatus 2 and following the USP711 protocol The pharmaceutically active ingredients rapidly and completely dissolve in aqueous solutions (using e.g. conditions: Apparatus 2, 50 rpm, pH 2,5). In one embodiment, after 30 minutes more than 80 % of the pharmaceutically active ingredient is dissolved, preferably more than 85 %, more preferably more than 90 %, even more preferably more than 95 %.

The compositions of the invention are further characterized by good stability results. Granulate prepared by described procedure has good flow properties which enables fast tablet compression with low tablet mass variation during production. Also friability of tablets is low which is Important for tablet film coating process.

In a preferred embodiment of the present invention a thiazolidinedione or a pharmaceutically acceptable salt thereof is formulated together with a biguanide as the second pharmaceutically active ingredient, preferably metformin, into a solid dosage form suitable for oral administration.

The term 'thiazolidinedione' in the meaning of the present invention relates to a class of insulin sensitizers and their pharmaceutically acceptable salts. Thiazolidinediones, which are also frequently referred to as glitazones, are known to reduce plasma glucose levels by increasing glucose uptake by liver, muscle or fat tissue via a mechanism that involves the nuclear receptor PPARγ (peroxisome proliferators-activated receptor, subtype γ). This class of compounds comprises, without being limited to, pioglitazone, rosiglitazone, troglitazone, rivoglitazone, englitazone and ciglitazone.

The term 'metformin' as used herein refers to its normal meaning as pharmaceutically active substance; the usual form is metformin hydrochlorid (metformin HCI).

In a preferred embodiment pioglitazone or a pharmaceutically acceptable salt thereof (e.g. pioglitazone hydrochloride) is formulated together with metformin. This is because the benefits of the present invention can be utilized effectively in case of this combination where usually the content of pioglitazone is substantially lower than that of metformin, and even more in the event that also the size pioglitazone particles is substantially lower than that of metformin particles which are originally used respectively. Yet, despite of these contstraints, metformin and pioglitazone are uniformly distributed in the final common dosage form, and allow rapid and complete dissolution of both active ingredient.

In accordance with a preferred embodiment, the present invention thus beneficially allows, in a robust, reliable and repeatable manner, to obtain immediate release granules and tablets comprising a combination of a relatively low content of thiazolidinedione or a pharmaceutically acceptable salt thereof and the second pharmaceutically active ingredient different from thiazolidinedione, such as a biguanide compound like metformin.

The amount of the thiazolidinedione such as pioglitazone per dosage unit is preferably in the range from 0.01 mg to 500 mg, more preferably from 1 mg to 100 mg, even more preferably from 2 mg to 20 mg. Owing to the concept of the present invention, the drug load of the thiazolidinedione per dosage unit can be kept low, if beneficially desired, for example 5 wt.-% of below, optionally 3 wt.-% or below and even 2 wt.-% or below.

The amount of the second pharmaceutically active ingredient different from thiazolidinedione is typically higher and can be substantially higher than the amount of the thiazolidinedione. For example, the advantages of the present invention can be particularly utilized when the amount of the second pharmaceutically active ingredient is 10-fold higher or more, or 25-fold higher or more, or even 50-fold higher or more.

The second pharmaceutically active ingredient, which is preferably metformin, can be present in amount suitably in the range from 100 mg to 3000 mg per dosage unit, preferably from 250 mg to 2500 mg per dosage unit.

In a particular embodiment dosage units comprise about 15 mg of pioglitazone together with either about 500 mg or about 850 mg of metformin. The term "about" is used to mean suitable tolerance of e.g. ± 10% of the respectively indicated value.

The granulation steps should be carried out by wet granulation. Preferably, the first granulation step is carried out in a high shear granulator, the second one in a fluid bed device.

The granulation mixture may comprise a suitable granulation liquid such as water, and optionally suitable pharmaceutical excipients. In preferred embodiments the granulation mixture comprises an excipient selected from the group consisting of povidone, hypromelose and hydroxypropyl cellulose. Excipient(s) for the first and second granulation respectively can be same or different. The respective granulation mixture contributes to enhanced tablet hardness.

In the case of wet granulation the obtained granulate can be dried, for example in a fluid bed device. The dried granulate may further optionally be sieved using, for example, an oscillating bar sieve or impact mill.

In one embodiment the granulate obtained in the second granulation step is further blended with suitable excipients, for example selected from the groups of fillers, binders, lubricants, surfactants, pigments, glidants and disintegrants, optionally also pigments, flavors, preservatives and the like. Preferable excipients include cellulose derivatives, croscarmellose sodium, magnesium stearate and/or other. The blending step may be carried out using, for example, a conventional bin blender.

In a preferred embodiment the composition according to the present Invention is a dosage form for oral administration, preferably a tablet or a tablet core. Tablets or tablet cores can be obtained by compression of the granulate obtained in the second granulation step, optionally together with suitable excipients such as, for example, fillers, binders, disintegrants, glidants, pigments, flavours and lubricants, including but not limited to microcrystalline cellulose, crosscarmelose sodium and magnesium stearate. Alternatively, the granulate comprising both active ingredients obtained in the second granulation step may also be filled in to sachets or capsules.

The tablets or tablet cores according to the invention may further comprise one or more coating layers. Such coating layers can e.g. be functional coatings such as film coatings, which for instance prevent environmental gases to ingress into the compartments. Further possible coatings are for instance coatings that improve the palatability or smoothness/gliding ability/sliding ability of the dosage form. Such optional one or more coating layers can comprise one or more of the excipients selected from the groups of pigments, flavors, binders, lubricants and glidants. In a preferred embodiment a coating layer is applied to the tablet cores that comprises hypromellose 2910, hydroxypropylcellulose, polyethylene glycol, titanium dioxide and talc. Excipients can be dissolved and dispersed in purified water and sprayed on the tablet cores, for instance in a perforated pan coating device.

The solid dosage form according to the present invention can be administered orally to mammals for prophylaxis or treatment of diabetes (e.g. type-1 diabetes, type-2 diabetes, gestational diabetes, etc.), diabetic complications (e.g. retinopathy, neuropathy, nephropathy, etc.) and all diseases linked to insuline-hyposensitivity.

### EXAMPLES

### EXAMPLE 1

### Tablet cores:

Tablet core composition:

| **component** | **amount per tablet (mg)** |
|---|---|
| pioglitazone hydrochloride | 16.534 |
| metformin hydrochloride | 850.000 |
| povidone | 54.466 |
| cellulose, microcrystalline | 114.000 |
| croscarmellose sodium | 50.000 |
| magnesium stearate | 5.000 |
| *purified water* *(removed during process)* | 218,000 |
| **TOTAL (TABLET CORES)** | **1090,000** |

The process for preparation of the tablet cores includes obtaining the granulate by a two-step granulation process, which comprises the following operations:
1. first granulation step including the granulation of pioglitazone hydrochloride (d0,5=4µm; d0,9=7µm), metformin hydrochloride (d0,9=327µm) and povidone (50% of total amount of povidone) in high shear granulator with purified water (8,3% of the total amount of water) and obtaining wet granulate 1;
2. drying of granulate 1 in fluid bed device;
3. sieving the dried granulate 1 with oscillating bar sieve,
4. second granulation step: granulation of dried and sieved granulate 1 in fluid bed device with a solution of povidone (50% of total amount) in purified water (91,7% of the total amount of water), and thereby obtaining wet granulate 2;
5. drying the wet granulate 2 in fluid bed device;
6. sieving the dried granulate 2 with oscillating bar sieve.

The obtained granulate 2 is then blended with microcrystalline cellulose and croscarmellose sodium in a bin blender. Pre-sieved magnesium stearate is added to the blend and blended in the bin blender in order to obtain the final blend.

The final blend is then compressed into tablet cores of 1090 mg using a rotary tableting machine.

### Coated tablets:

**Coated tablet composition:**

| **component** | **amount per tablet (mg)** |
|---|---|
| tablet cores | 1090,000 |
| hypromellose 2910 | 17.200 |
| hydroxypropylcellulose | 2.000 |
| polyethylene glycol 6000 | 3.000 |
| titanium dioxide | 6.300 |
| talc | 1.500 |
| *purified water* *(removed during process)* | 255.000 |
| *talc* *(not included in the final sum)* | 0.330 |
| **TOTAL (TABLET COATING)** | **30.000** |
| **TOTAL (FILM COATED TABLETS)** | **1120.000** |

The process for preparation of coated tablets comprises:
1. preparation of the coating dispersion by dissolving and dispersing hypromellose, hydroxypropylcellulose, polyethylene glycol, titanium dioxide talc in purified water;
2. spraying the coating dispersion onto the tablet cores in a perforated pan coating device;
3. drying and cooling down coated tablets;
4. polishing coated tablets with talc.

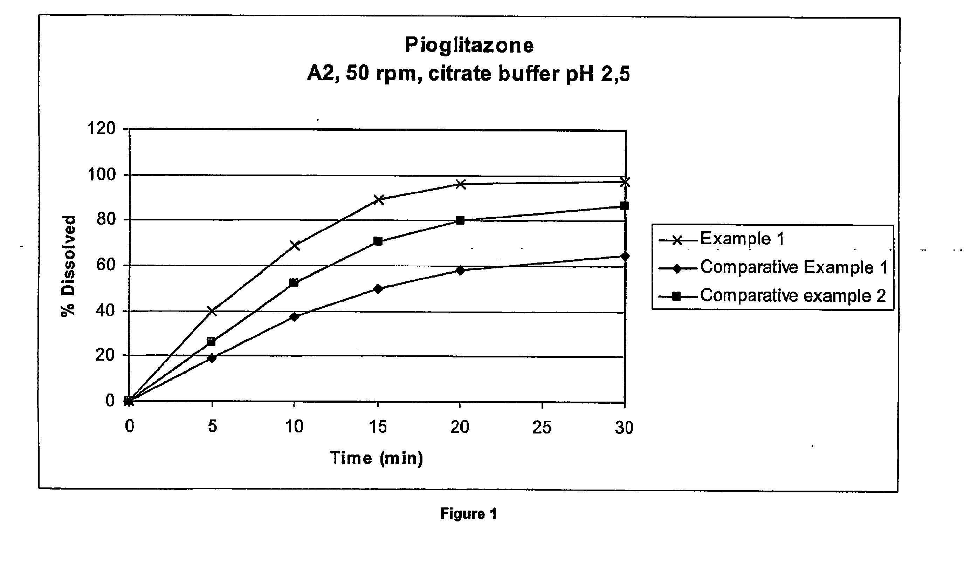

The obtained coated tablets are characterized by an appropriate dissolution rate of pioglitazone HCI as shown in Figure 1.

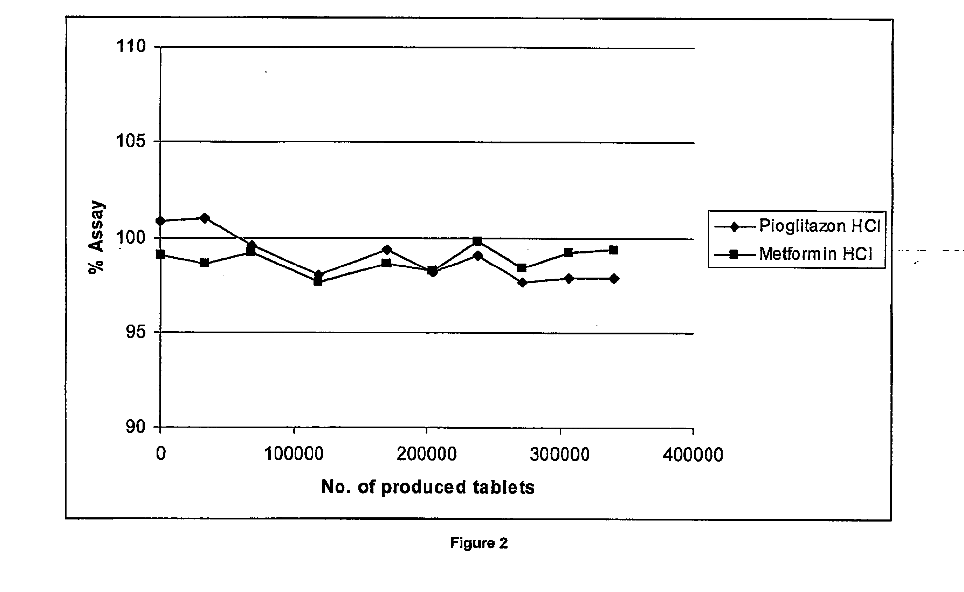

The tableting mixture of Example 1 efficiently avoids segregation tendency and therefore assures appropriate content uniformity of obtained tablets (Figure 2).

### EXAMPLE 2

### Tablet cores:

**Tablet core composition:**

| **component** | **amount per tablet (mg)** |
|---|---|
| pioglitazone hydrochloride | 16.534 |
| metformin hydrochloride | 500.000 |
| povidone | 32.466 |
| cellulose, microcrystalline | 68.200 |
| croscarmellose sodium | 29.800 |
| magnesium stearate | 3.000 |
| *purified water (removed during process)* | 130,000 |
| **TOTAL (TABLET CORES)** | **650,000** |

The process for preparation of the tablet cores includes obtaining the granulate by a two-step granulation process which contains next operations:
1. first granulation step including granulation of pioglitazone hydrochloride (d0,5=3µm; d0,9=7µm), metformin hydrochloride (d0,9=299µm), and povidone (50% of total amount of povidone) in high shear granulator with purified water (8,3% of the total amount of water) and obtaining wet granulate 1;
2. drying of granulate 1 in fluid bed device;
3. sieving dried granulate 1 with oscillating bar sieve,
4. second step of granulation including granulation of dried sieved granulate 1 in fluid bed device with the solution of povidone (50% of total amount) in purified water (91,7% of the total amount of water), and thereby obtaining wet granulate 2;
5. drying the wet granulate 2 in fluid bed device;
6. sieving the dried granulate 2 with oscillating bar sieve.

The obtained granulate 2 is then blended with microcrystalline cellulose and croscarmellose sodium in a bin blender. Sieved magnesium stearate is added to the blend and blended in the bin blender in order to obtain the final blend.

The final blend is then compressed into tablet cores of 650 mg on a rotary tableting machine.

### Coated tablets:

Coated tablet composition:

| **component** | **amount per tablet (mg)** |
|---|---|
| tablet cores | 650,000 |
| hypromellose 2910 | 10.320 |
| hydroxypropylcellulose | 1.200 |
| polyethylene glycol 6000 | 1.800 |
| titanium dioxide | 3.780 |
| talc | 0.900 |
| *purified water* *(removed during process)* | 152.000 |
| *talc* *(not included in the final sum)* | 0.200 |
| **TOTAL (TABLET COATING)** | **30.000** |
| **TOTAL (FILM COATED TABLETS)** | **1120.000** |

The process for preparation of coated tablets comprises:
1. preparation of the coating dispersion by dissolving and dispersing hypromellose, hydroxypropylcellulose, polyethylene glycol, titanium dioxide talc in purified water;
2. spraying the coating dispersion onto the tablet cores in a perforated pan coating device;
3. drying and cooling down coated tablets;
4. polishing coated tablets with talc.

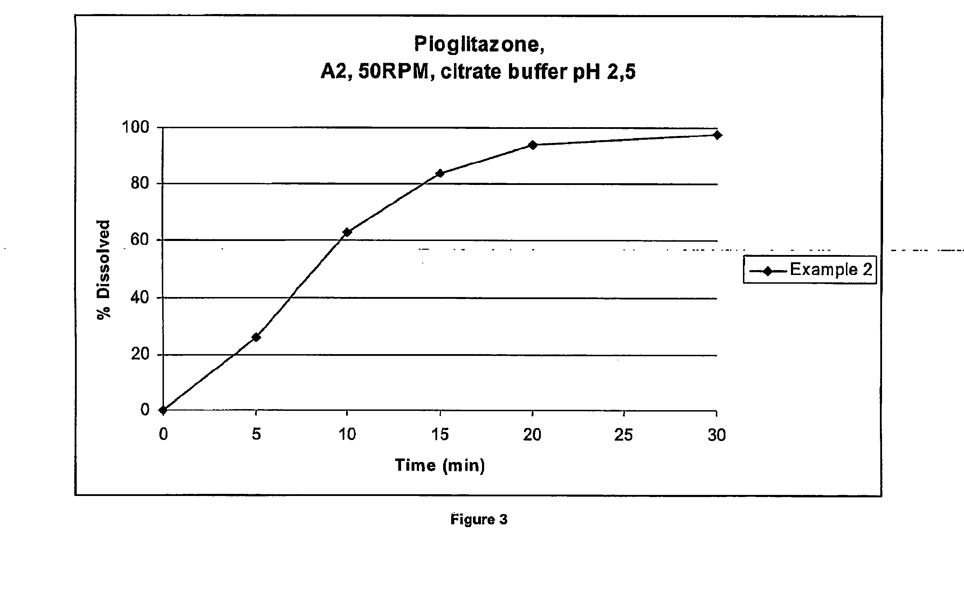

The dissolution rate of the tablets of Example 2 is shown in Figure 3.

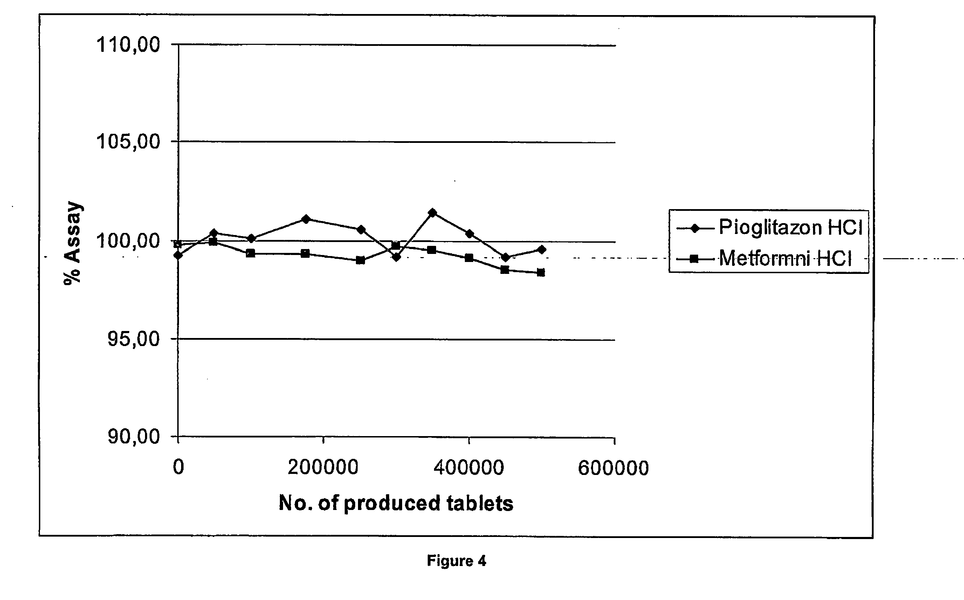

The tableting mixture of Example 2 further shows no segregation tendency and therefore assures appropriate content uniformity of obtained tablets (Figure 4).

### COMPARATIVE EXAMPLE 1

### Tablet cores:

Tablet core composition:

| **component** | **amount per tablet (mg)** |
|---|---|
| pioglitazone hydrochloride | 16.534 |
| metformin hydrochloride | 850.000 |
| povidone | 54.466 |
| cellulose, microcrystalline | 105.700 |
| croscarmellose sodium | 50.000 |
| magnesium stearate | 5.000 |
| *purified water* *(removed during process)* | 500,000 |
| **TOTAL (TABLET CORES)** | **1080,000** |

The process for preparation of the tablet cores includes obtaining the granulate by fluid bed granulation process which comprises next operations:
1. dissolving of povidone in purified water (in 80% of the total amount of water);
2. dispersing pioglitazone hydrochloride (d0,5=3µm; d0,9=7µm) in 20% of the total amount of water purified water;
3. joining the povidone solution and pioglitazone hydrochloride suspension and obtaining the granulation liquid;
4. granulation of metformin hydrochloride (d0,9=306µm) in a fluid bed device with prepared granulation liquid, and thereby obtaining wet granulate;
5. drying the wet granulate in fluid bed device;
6. sieving the dried granulate with oscillating bar sieve.

The obtained granulate is blended with microcrystalline cellulose and croscarmellose sodium in a bin blender. Sieved magnesium stearate is added to the blend and blended in the bin blender so that a final blend is obtained.

The final blend is then compressed into tablet cores of 1080 mg on a rotary tableting machine.

Figure 1 shows that the dissolution rate of pioglitazone in Comparative (Example 1 is significantly slower than in Example 1. Extrapolation of the curve in Figure 1 furthermore indicates that pioglitazone is not completely dissolved in Comparative Example 1.

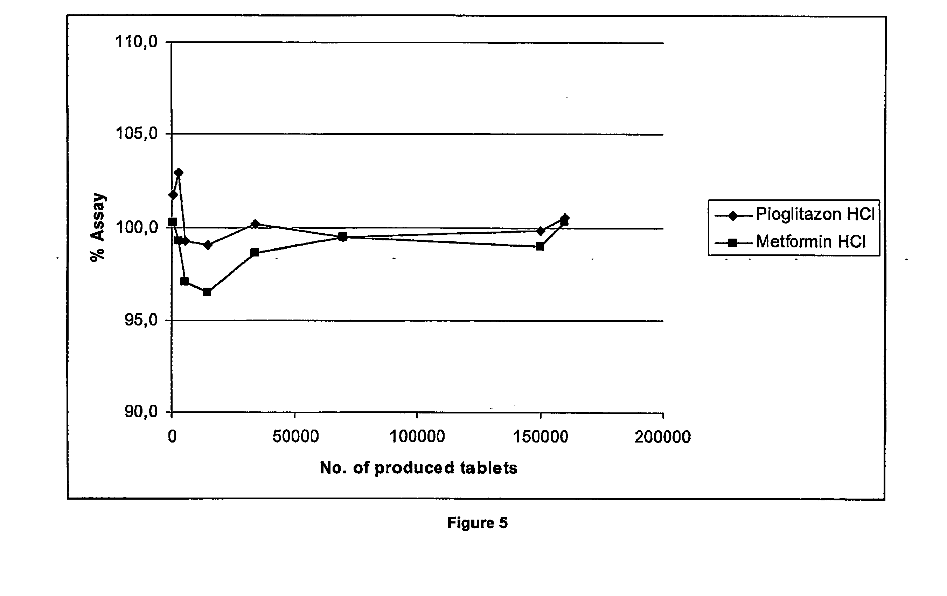

However, no seggregation tendency can be observed in the composition according to Comparative Example 1, which indicates appropriate content uniformity (Figure 5).

### COMPARATIVE EXAMPLE 2

### Tablet cores:

**Tablet core composition:**

| **component** | **amount per tablet (mg)** |
|---|---|
| pioglitazone hydrochloride | 16.534 |
| metformin hydrochloride | 850.000 |
| povidone | 54.466 |
| cellulose, microcrystalline | 114.000* |
| croscarmellose sodium | 50.000 |
| magnesium stearate | 5.000 |
| *purified water* *(removed during process)* | 48,000 |
| **TOTAL (TABLET CORES)** | **1090,000** |

The process for preparation of the tablet cores includes granulate preparation which comprises the following steps:
1. granulation of pioglitazone hydrochloride(d0,5=3µm; d0,9=7µm), metformin hydrochloride (d0,9=327µm) and povidone in high shear granulator with purified water;
2. drying of granulate of step 1 in fluid bed device;
3. sieving the dried granulate with oscillating bar sieve,

The obtained granulate is blended with microcrystalline cellulose and croscarmellose sodium in a bin blender. Sieved magnesium stearate is added to the blend and blended in the bin blender in order to obtain a final blend.

The final blend is then compressed into tablet cores of 1090 mg on a rotary tableting machine.

The tablets obtained in this manner are characterized by a dissolution rate, which is faster than that of Comparative Example 1, but still considerably slower than that of Example 1 (Figure 1).

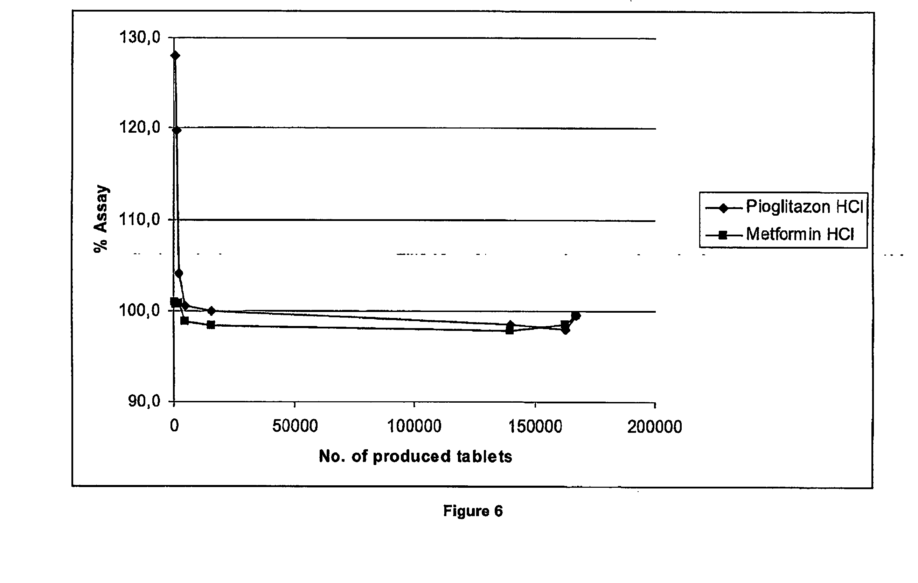

In addition, the tableting mixture shows segregation tendency and therefore does not assure appropriate content uniformity of obtained tablets (Figure 6).

### EXAMPLE 3 AND COMPARATIVE EXAMPLES 3 AND 4

Pioglitazon/Metformin tablets were prepared. A pigment was respectively added for the purpose to detect differences in structure after tablet compression after different techniques, i.e. the two-step granulation technique according to the present invention and comparative techniques. It shows how the two-step granulation disclosed herein is reflected in granulate structure, and its consequence of enabling good active ingredient homogeneity, dissolution and physical properties. While such pigment addition may be regarded as a reference to evaluate the resulting structure, other methods to identify the structure may alternatively be used without different coloring of first and second granulate, such as Raman spectroscopic methods, further microscopic methods such as electron microscopy, without being limited thereto.

Tablets were prepared by three different techniques:
a) dry mixture of ingredients: all ingredients were dry mixed together, sieved and compressed into tablets (Comparative Example 3)
b) high-shear granulation was preformed (Comparative Example 4) as described in comparative example 2
c) two step granulation was preformed (Example 3) as described in Example 1.

To detect granulate structure 0.09 % of total tablet mass of black pigment was added to Pioglitazone before sample preparation. Prepared tablets were tested on hardness, prolonged friability and disintegration. Also pictures were taken using light microscope to study granulate structure.

The physical characteristics of the prepared samples from a, b and c are shown below:

| | hardness * | prolonged friability | disintegration |
|---|---|---|---|
| sample a | 20 N | >10% | 40 seconds |
| sample b | 100 N | >10% | 1.5 minutes |
| sample c | 190 N | 0.47 % | 7 minutes |

| | | | |
|---|---|---|---|
| * tablets were compressed with 20kN force | | | |

Sample c gave optimal results, whereas sample a and b did not have acceptable physical caracteristics.

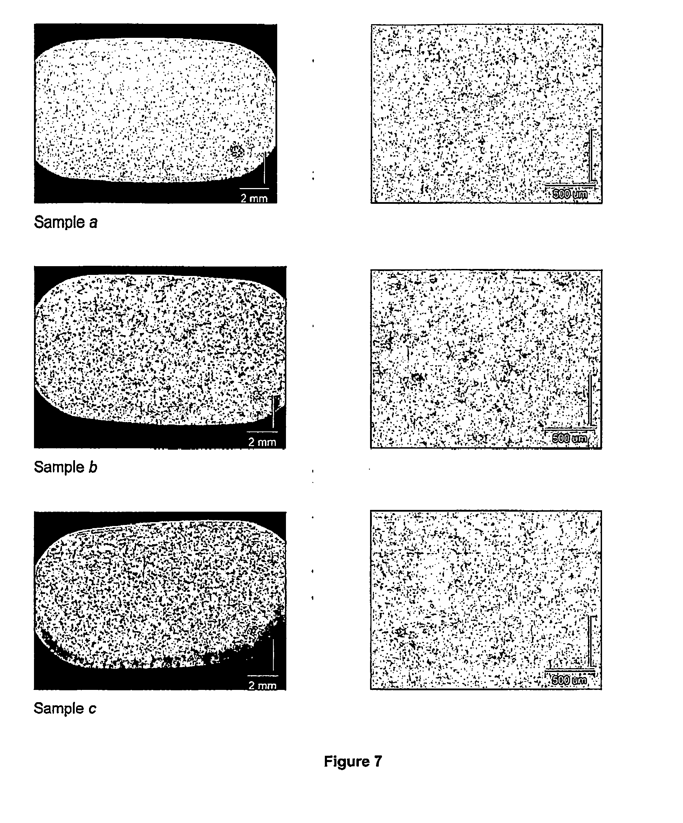

Moreover, microscopic analysis of each of a, b and c are shown in Fig. 7. Observing tablet morphology, it is clearly visible that sample b is least homogenous as there are parts of tablet with more pigment particles in larger spots and parts with less pigment. Sample a is the most homogenous, but as shown above it has very poor physical characteristics. On Sample c the granulation effect is observed but in contrast to sample b granulate is homogenously dispersed throughout the tablet, combined with excellent physical properties..

Therefore, it is concluded that:
- Tableting mixture preparation has a large effect on Pioglitazon/Metformin tablet physical characteristics - two-step granulation being the best option.
- Tableting mixture preparation has effect on particle/granulate dispersion in tablet. Two-step granulation resulted in more homogenous tablets in comparison with one step granulation.

## Claims

1. A pharmaceutical composition comprising a combination of thiazolidinedione or a pharmaceutically acceptable salt thereof as a first pharmaceutically active ingredient and a second pharmaceutically active ingredient different from thiazolidinedione, wherein the amount of said second pharmaceutically ingredient is larger than that of the first pharmaceutically active ingredient, wherein the combination of said first and second pharmaceutically active ingredients are provided by
a first granulate comprising the first and second pharmaceutically active ingredients and optionally at least one excipient, said first granulate being present in a second granulate comprising a further pharmaceutical excipient.

2. The pharmaceutical composition according to claim 1, wherein the combination of said first and second pharmaceutically active ingredients is obtainable by subjecting said first granulate to granulation to form said second granulate comprising said first granulate, and the second granulate is formulated into a solid dosage form.

3. The pharmaceutical composition according to claim 1 or 2, which is in the form of tablets or tablet cores.

4. The pharmaceutical composition according to claim 1 or 2, which is in the form of the second granulate, which comprises the first granulate comprising the first and second pharmaceutically active ingredients, and which second granulate is filled into capsules or sachets.

5. The pharmaceutical composition according to claim 3, wherein the tablets or tablet cores comprise a coat.

6. The pharmaceutical composition according to claim 5, wherein the coat comprises one or more substances selected from the group consisting of hydroxpropylcellulose, polyethylene glycol, titanium dioxide and talc.

7. The pharmaceutical composition according to any of the preceding claims, wherein the pharmaceutically active ingredient different from thiazolidinedione is a biguanide compound, preferably metformin hydrochloride.

8. The pharmaceutical composition according to any of the preceding claims, wherein the thiazolidinedione is pioglitazone or a pharmaceutically active salt thereof.

9. The pharmaceutical composition according to claim 3 in the form of tablets or tablet cores, wherein both the first and second pharmaceutically active ingredients are present in said first and second granulates, and by tabletting the second granulate isniformly distributed throughout each tablet or tablet core.

10. The pharmaceutical composition according to any of the preceding claims, wherein the drug load of the thiazolidinedione per dosage unit is 5 wt.-% of below, optionally 3 wt.-% or below.

11. The pharmaceutical composition according to any of the preceding claims, wherein the amount of the second pharmaceutically active ingredient is 10-fold higher or more, optionally 25-fold higher or more than the amount of the thiazolidinedione.

12. The pharmaceutical composition according to any of the preceding claims, wherein the ratio of median size of the thiazolidinedione compound, preferably when the thiazolidinedione compound is pioglitazone hydrochloride, to the median size of the second pharmaceutically active ingredient, preferably when it is metformin hydrochloride, is higher than 15.

13. The pharmaceutical composition according to any of the preceding claims, which comprises povidone, hypromelose and/or hydroxypropyl cellulose as a binder, preferably wherein povidone has been used for both the first and the second granulate.

14. The pharmaceutical composition according to any of the preceding claims, wherein the *in vitro* dissolution rate of pioglitazone as determined by USP 711 is such that after 30 minutes, preferably after 20 minutes, more than 80 % of the total amount of pioglitazone is dissolved.

15. The pharmaceutical composition according to any of the preceding claims, **characterized by** a hardness higher than 200N and friability below 0,2% at 15/850 strength, and hardness higher than 150N and friability below 0,2% at 15/500 strength.

16. A process for manufacturing a pharmaceutical composition comprising a combination of thiazolidinedione or a pharmaceutically acceptable salt thereof as a first pharmaceutically active ingredient and a second pharmaceutically active ingredient different from thiazolidinedione, the process comprising the steps of
a) granulation of the first and second pharmaceutically active ingredients together with at least one suitable excipient to obtain a first granulate, and
b) subsequent granulation of the first granulate of step a), together with the same or different at least one pharmaceutical excipient.

17. The pharmaceutical composition according to any of claims 1 to 15 for use as a medicament for prophylaxis or treatment of diabetes and/or diabetic complications.
